# EUROPÄISCHE PATENTSCHRIFT

(11) **EP 2 328 544 B1**
(45) Veröffentlichungstag und Bekanntmachung des Hinweises auf die Patenterteilung: **16.09.2015**
(21) Anmeldenummer: 09780312.6
(22) Anmeldetag: 08.07.2009
(51) Int. Cl.: A61K 8/39, A61K 8/86, A61K 8/92, A61Q 5/02, A61Q 5/12, A61Q 19/10, B01F 17/00

(54) **TENSIDHALTIGE ZUSAMMENSETZUNG MIT SPEZIELLER EMULGATORMISCHUNG**
COMPOSITION CONTAINING SURFACTANTS AND HAVING A SPECIAL EMULSIFIER MIXTURE
COMPOSITION À BASE DE TENSIOACTIFS CONTENANT UN MÉLANGE D'ÉMULSIFIANTS SPÉCIAL

(30) Priorität: 23.07.2008 DE 102008034388
(43) Veröffentlichungstag der Anmeldung: 08.06.2011
(73) Patentinhaber: Henkel AG & Co. KGaA, 40589 Düsseldorf (DE)
(72) Erfinder: SARTINGEN, Kirsten, 41379 Brüggen (DE)
(86) Internationale Anmeldenummer: PCT/EP2009/058665
(87) Internationale Veröffentlichungsnummer: WO 2010/009978

(56) Entgegenhaltungen:
- WO-A1-2005/048971
- DE-A1-102005 052 100
- DE-A1-102006 030 092
- Datenblatt zu dem Handelsprodukt "Lösungsvermittler 660352®"

## Beschreibung

Die vorliegende Erfindung betrifft eine Zusammensetzung auf Tensidbasis, in der mithilfe einer speziellen Emulgatormischung eine oder mehrere Ölkomponenten stabil suspendiert wurden, sowie die Verwendung der Zusammensetzung als kosmetisches Mittel.
Des weiteren betrifft die vorliegende Erfindung ein Verfahren zur Suspendierung von Ölkomponenten in einer Tensidmischung.

Reinigungsmittel, beispielsweise kosmetische Reinigungsmittel für die Haut und die Haare wie flüssige Seifen, Shampoos, Duschbäder, Schaumbäder, Dusch- und Waschgele, müssen nicht nur ein gutes Reinigungsvermögen aufweisen, sondern sollen weiterhin für die Haut und die Schleimhäute gut verträglich sein und auch bei häufiger Anwendung nicht zu starker Entfettung oder Hauttrockenheit führen.
Aus diesem Grund wird seit vielen Jahren versucht, möglichst viele Haar- und Hautkonditioniermittel in die Reinigungsmittel einzuarbeiten - einerseits, um den Anforderungen hinsichtlich der gleichzeitigen Reinigung und Pflege entgegen zu kommen und andererseits, damit der Verbraucher durch die Anwendung nur noch eines Produkts, das diese beiden Anforderungen erfüllt, sowohl den Vorteil der Zeitersparnis, als auch den Vorteil der Kostenersparnis genießen kann (da optimalerweise ein Pflegeschritt mit einem Haut- oder Haarnachbehandlungsmittel überflüssig werden soll).
Die Einarbeitung von Haut- und Haarkonditioniermittel in eine tensidische Reinigungsformulierung stellt die Hersteller solcher Zusammensetzungen aber bis auf den heutigen Tag immer wieder vor große Schwierigkeiten, denn insbesondere mineralische, natürliche oder synthetische Fett-, Wachs- und Ölkomponenten, die nachweislich einen pflegenden und konditionierenden Effekt auf der Haut und den Haaren hinterlassen, lassen sich nicht durch einfaches Einmischen in eine Tensidbasis einarbeiten.
So war man in den vergangenen Jahren dazu gezwungen beispielsweise Perlglanzmittel, spezielle Polymere und/oder Salze in kosmetische Reinigungsmittel einzuarbeiten, damit man beispielsweise Silikone lagerstabil in den Formulierungen suspendieren konnte.
In neuester Zeit geht der Trend nun wieder hin zu natürlichen kosmetischen Produkten auf der Basis milder (möglichst biologisch abbaubarer) Tenside, die natürliche Pflegestoffe pflanzlichen Ursprungs enthalten. In solchen Produkten soll sowohl die Zahl nicht unbedingt notwendiger Wirkstoffe, die ein Irritationspotential auf der Haut bzw. der Kopfhaut hervorrufen, minimiert werden, als auch bei den notwendigen Komponenten streng auf Hautverträglichkeit, Milde und biologische Abbaubarkeit geachtet werden.
Somit stellte sich wieder eine vollkommen neue Problematik, denn eine natürliche Ölkomponente mit möglichst wenigen chemischen Hilfsstoffen in einer milden Tensidbasis zu stabilisieren, war nicht trivial. Zum einen war es nicht immer möglich, klare Formulierungen herzustellen (die aber vom Verbraucher gewünscht werden, da sie mit natürlichen Zusammensetzungen ohne "Chemie" assoziiert werden) und zum anderen war die Einarbeitung der natürlichen Öle in die Tensidbasis meist mit großen Problemen hinsichtlich der stabilen Suspendierung über einen längeren Zeitraum und bei Temperaturschwankungen und mit einem dramatischen Viskositätsabfall der Zusammensetzungen verbunden.

Ziel der vorliegenden Erfindung war es daher eine klares Reinigungsmittel auf Tensidbasis herzustellen, in dem eine Ölkomponente (optimalerweise eine pflanzliche Ölkomponente) über einen langen Zeitraum und bei Temperaturschwankungen stabil suspendiert werden kann.
Ein weiteres Ziel der Erfindung war es ein klares Reinigungsmittel herzustellen, das eine ausreichend hohe Viskosität aufweist, damit es einfach aus dem Behälter appliziert werden kann, ohne zu "verlaufen" und zu tropfen. Dabei soll die Anfangsviskosität bei der Lagerung möglichst erhalten bleiben.

Diese Ziele wurden in hohem Maße erreicht, indem eine spezielle Emulgatormischung gefunden wurde, die in einem speziellen Mischungsverhältnis in der Tensidbasis vorliegen muss.
Mithilfe dieser Emulgatormischung war es möglich eine Vielzahl von Ölkomponenten stabil in eine (milde) Tensidbasis einzuarbeiten und es wurde gefunden, dass auch bei einer Lagerung bei hohen Temperaturen (50 bzw. 60°C) die Suspendierungswirkung erhalten blieb und die Viskosität der Zusammensetzung sich nicht veränderte.
Es war möglich, die Emulatormischung in allen gängigen Tensidsystemen einzusetzen - insbesondere aber war es möglich, eine milde Tensidbasis und pflanzliche Öle auszuwählen - und dennoch die erfindungsgemäßen Ziele zu erfüllen. Damit eignen sich die klaren, erfindungsgemäßen Zusammensetzungen hervorragend als kosmetische Reinigungsmittel für die Haut und die Haare, denn sie erfüllen die vorgenannten Bedürfnisse der Verbraucher nach milden, natürlichen Produkten.
Gegenstand der vorliegenden Erfindung ist daher eine klare tensidhaltige Zusammensetzung, die neben einer oder mehrerer Ölkomponente(n) ein Gemisch aus Lösungsvermittlern enthält, die ausgewählt sind aus der Gruppe der
a) ethoxylierten Fettalkohole,
b) ethoxylierten hydrierten Rizinusöle und
c) ethoxylierten Mono-, Di- oder Triglycerinester,
wobei das Verhältnis der Komponenten a) : b) : c) im Bereich von 1 : (2-4): (3-4) liegt.

Unter einer klaren Zusammensetzung wird erfindungsgemäß eine Zusammensetzung verstanden, die einen NTU-Wert (Nephelometric Turbidity Unit = Nephelometrischer Trübungswert) ≤ 50, bevorzugt ≤ 30, besonders bevorzugt ≤ 20 uns insbesondere ≤ 10 aufweist.

Die ersten zwingende Komponente a) der erfindungsgemäßen Emulgatormischung ist ein ethoxylierter Fettalkohol, bei dem es sich um einen geradkettigen oder einen verzweigten, gesättigten oder ungesättigten C₈-C₂₂-Fettalkohol, bevorzugt um einen C₁₀-C₂₀-Fettalkohol, besonders bevorzugt um einen C₁₂-C₁₈-Fettalkohol und insbesondere um einen C₁₃-C₁₅-Fettalkohol mit einem Ethoxylierungsgrad von 1 bis 18, bevorzugt von 5 bis 16, besonders bevorzugt von 7 bis 14 und insbesondere von 8 bis 12, handelt.
Erfindungsgemäß bevorzugte Komponenten a) sind beispielsweise Deceth-5, Deceth-7, Undeceth-7, Laureth-4, Laureth-5, Laureth-6, Laureth-7, Laureth-8, Laureth-9, Laureth-10, Laureth-12, Trideceth-5, Trideceth-7, Trideceth-8, Trideceth-9, Trideceth-10, Triedceth-12, Myreth-8, Myreth-10 und Myreth-12. Besonders bevorzugt sind Laureth-7, Laureth-8, Laureth-9, Laureth-10, Trideceth-5, Trideceth-7, Trideceth-8, Trideceth-9, Trideceth-10 und insbesondere bevorzugt ist Trideceth-9.

Komponente b) ist erfindungsgemäß ethoxyliertes hydriertes Rizinusöl mit einem Ethoxylierungsgrad von 5 bis 80, bevorzugt von 10 bis 60, besonders bevorzugt von 25 bis 50 und insbesondere von 35 bis 45.
Besonders geeignet sind PEG-10 Hydrogenated Castor Oil, PEG-25 Hydrogenated Castor Oil, PEG-40 Hydrogenated Castor Oil und PEG-60 Hydrogenated Castor Oil, insbesondere geeignet ist PEG-40 Hydrogenated Castor Oil, wie es beispielsweise unter dem Handelsnamen Cremophor^{®} RH 410 oder Eumulgin^{®} HRE 455 im Handel erhältlich ist.
Erfindungsgemäß ist es auch möglich, die Komponenten a) und b) in einer Mischung einzusetzen. Derartige, sogenannte "Solubilizer"-Mischungen sind im Handel beispielsweise unter der Bezeichnung Solubilizer^{®} 611674 oder Solubilizer^{®} 660352 erhältlich.

Die dritte essentielle Komponente der erfindungsgemäßen Emulgatormischung ist ein Monoester und/oder ein Gemischen aus Monoestern und Diestern des Glycerins mit verzweigten, oder geradkettigen, gesättigten oder ungesättigten Fettsäuren einer C-Kettenlänge von 8 bis 24, bevorzugt von 10 bis 18 und insbesondere von 12 bis 16, die einen Ethoxylierungsgrad von 1 bis 20, bevorzugt von 2 bis 17, besonders bevorzugt von 4 bis 13 und insbesondere von 6 bis 10 aufweisen.
Erfindungsgemäß bevorzugt sind die ethoxylierten Glyceryloleate und Glycerylcocoate und insbesondere bevorzugt ist PEG-7 Glyceryl Cocoate, wie es beispielsweise im Handel unter der Bezeichnung Tegosoft^{®} GC oder Cetiol^{®} HE erhältlich ist.
Die drei Emulgatoren a), b) und c) werden in der erfindungsgemäßen Zusammensetzung - bezogen auf ihr Gesamtgewicht -jeweils in einer Menge von 0,05 bis 3 Gew.-%, bevorzugt von 0,1 bis 2 Gew.-% und insbesondere von 0,2 bis 1,5 Gew.-% eingesetzt.

Zur Erfüllung des erfindungsgemäßen Ziels der Viskositätsstabilität weisen die erfindungsgemäßen Zusammensetzungen eine Viskosität im Bereich von 5,000 bis 9,500 mPas, bevorzugt von 6,000 bis 9,000 mPas und insbesondere von 7,000 bis 8,000 mPas (jeweils gemessen mit einem Haake Rotationsviskosimeter VT550; 20°C; Meßeinrichtung MV; Spindel MV II; 8 UPM) auf. Zusammensetzungen dieser Viskosität lassen sich bequem und einfach aus einem Behälter auf die Hand oder die Anwendungsoberfläche applizieren, ohne zwischen den Fingern hindurchzulaufen und zu tropfen. Gleichzeitig ist die Viskosität der Zusammensetzung aber auch niedrig genug, damit eine zufriedenstellende Verteilung auf der Anwendungsoberfläche unter Zuhilfenahme der Hände gewährleistet ist.

Der pH-Wert der erfindungsgemäßen Zusammensetzungen liegt idealerweise in einem hautschonenden Bereich von etwa 3 bis 7, bevorzugt in einem Bereich von 4 bis 6,5 und insbesondere in einem Bereich von 4,5 bis 5,5.

Ein weiterer wesentlicher Bestandteil der erfindungsgemäßen Zusammensetzungen ist eine oder mehrere Ölkomponente(n).
Diese kann (können) ausgewählt sein aus mineralischen, natürlichen oder synthetischen Ölkomponenten wie Petrolatum, Paraffinen, Silikonen, Fettalkoholen, Fettsäuren, Fettsäureestern sowie natürlichen Ölen pflanzlichen und tierischen Ursprungs, und werden in den Zusammensetzungen - bezogen auf ihr Gesamtgewicht - in einer Menge von 0,005 bis 20 Gew.-%, bevorzugt von 0,01 bis 10 Gew.-%, besonders bevorzugt von 0,05 bis 5 Gew.-% und insbesondere von 0,2 bis 3 Gew.-% eingesetzt.

Unter dem Begriff Silikonöle versteht der Fachmann mehrere Strukturen siliciumorganischer Verbindungen.
Insbesondere bevorzugt werden die Silikone ausgewählt, aus mindestens einem Vertreter aus der siliciumorganischer Verbindungen, die gebildet werden aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nicht silikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Gemischen.

In einer Ausführungsform der vorliegenden Erfindung ist das Konditioniermittel ein konditionierendes Silikon mit einer Viskosität von 20.000 bis 120.000 mPa·s, ganz besonders bevorzugt von 40.000 bis 80.000 mPa·s.
Besonders bevorzugt ist dabei das konditionierende Silikon ausgewählt aus Dimethiconen, Amodimethiconen oder Dimethiconolen.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.
Die Einsatzmenge beträgt dabei 0,1 - 15 Gew.-%, bezogen auf das gesamte Mittel. Bevorzugt beträgt die Menge 0,5 - 10 Gew.-%, wobei ganz besonders vorteilhaft Mengen von 1 - 5 Gew.-% sein können.

Als Fettalkohole können eingesetzt werden gesättigte, ein- oder mehrfach ungesättigte, verzweigte oder unverzweigte Fettalkohole mit C₆-C₃₀-, bevorzugt C₁₀-C₂₂- und ganz besonders bevorzugt C₁₂-C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 -20 Gew.-% eingesetzt.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.
Die Einsatzmenge beträgt 0,1 - 50 Gew.-% bezogen auf das gesamte Mittel, bevorzugt 0,1 - 20 Gew.-% und besonders bevorzugt 0,1 - 15 Gew.-% bezogen auf das gesamte Mittel.

Zu den natürlichen und synthetischen kosmetischen Ölkörpern sind beispielsweise zu zählen:
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Unter Esterölen sind zu verstehen die Ester von C₆-C₃₀ - Fettsäuren mit C₂-C₃₀-Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C₁₆-₁₈-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).

- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-dipelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel, 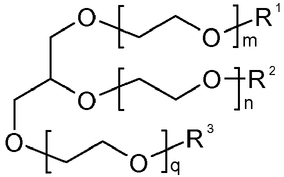 in der R1, R2 und R3 unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R1 für einen Acylrest und R2 und R3 für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

In einer besonders bevorzugten Ausführungsform der Erfindung wir als Ölkomponente ein pflanzliches Öl eingesetzt.
Als natürliche Öle kommen beispielsweise Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Hagebuttenkernöl, Haselnussöl, Holundersamenöl, Johannisbeersamenöl, Jojobaöl, Kakaobutter, Leinöl, Macadamianussöl, Maiskeimöl, Mandelöl, Marulaöl, Nachtkerzenöl, Olivenöl, Palmöl, Pfirichkernöl, Rapsöl, Reisöl, Sanddornfruchtfleischöl, Sanddornkernöl, Sesamöl, Sheabutter, Sojaöl, Sonnenblumenöl, Traubenkernöl, Walnußöl oder Wildrosenöl in Frage. Besonders bevorzugt sind erfindungsgemäß Avocadoöl, Aprikosenkernöl, Hagebuttenkernöl, Jojobaöl, Kakaobutter, Mandelöl, Olivenöl, Pfirichkernöl, Sheabutter, Sonnenblumenöl und Traubenkernöl.
Die pflanzlichen Öle werden in den erfindungsgemäßen Mitteln - bezogen auf das Gesamtgewicht der Mittel - in einer Menge von 0,001 bis 10 Gew-%, bevorzugt von 0,005 bis 7 Gew.-%, besonders bevorzugt von 0,01 bis 5 Gew.-% und insbesondere von 0,05 bis 3 Gew.-% eingesetzt.

Neben der Emulgatormischung und der Ölkomponente ist die Tensidbasis die dritter essentielle Komponente der erfindungsgemäßen Zusammensetzung.
Unter dem Begriff Tenside werden oberflächenaktive Substanzen verstanden, welche im Molekül eine anionische oder kationische Ladung, oder eine anionische und eine kationische Ladung tragen. Weiterhin können die oberflächenaktiven Substanzen auch nicht-ionisch sein.
Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (TI), 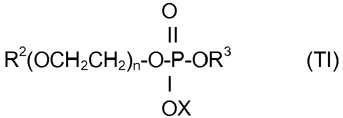 in der R²⁹ bevorzugt für einen aliphatischen Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄ - Kohlenwasserstoffrest, steht,
- sulfatierte Fettsäurealkylenglykolester der Formel (TII)

   R³⁵CO(AlkO)ₙSO₃M (TII)

   in der R³⁵CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (TIII), 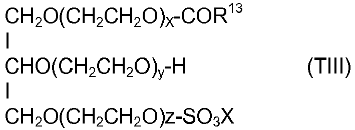 in der R¹³CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze.
Vorzugsweise werden Monoglyceridsulfate der Formel (TIII) eingesetzt, in der R³⁶CO für einen linearen Acylrest mit 8 bis 18 Kohlenstoffatomen steht.
Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO⁽⁻⁾ - oder-SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈- Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel R³⁷CO-(OCH₂CHR³⁸)_{w}OR³⁹, (TIV), in der R³⁷CO für einen linearen oder verzweigten, gesättigten und/oder ungesättigten Acylrest mit 6 bis 22 Kohlenstoffatomen, R³⁸ für Wasserstoff oder Methyl, R³⁹ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.
Eine weitere Gruppe erfindungsgemäß geeigneter, nichtionischer Tenside sind die Alkylpolyglucoside. Sie entsprechen der Formel (I)

R¹O-[G]ₚ (I)

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hierauf die Schriften EP-A1-301 298 und WO 90/03977 A1 verwiesen. Die Alkyl- und/oder Alkenyloligoglycoside können sich von Aldosen bzw. Ketosen mit 5 oder 6 Kohlenstoffatomen, vorzugsweise der Glucose ableiten. Die bevorzugten Alkyl- und/oder Alkenyloligoglycoside sind somit Alkyl- und/oder Alkenyloligoglucoside. Die Indexzahl p in der allgemeinen Formel (I) gibt den Oligomerisierungsgrad (DP), d.h. die Verteilung von Mono- und Oligoglycosiden anund steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglycosid eine analytisch ermittelte rechnerische Größe, die meistens eine gebrochene Zahl darstellt. Vorzugsweise werden Alkyl- und/oder Alkenyloligoglycoside mit einem mittleren Oligomerisierungsgrad p von 1,1 bis 3,0 eingesetzt. Aus anwendungstechnischer Sicht sind solche Alkyl- und/oder Alkenyloligoglycoside bevorzugt, deren Oligomerisierungsgrad kleiner als 1,7 ist und insbesondere zwischen 1,2 und 1,7 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylerstern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1-3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}Oxoalkohole (DP = 1-3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylakohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucyalkohol, Brassidylalkohol sowie deren technsiche Gemische, wie sie wie oben beschrieben erhalten werden können. Erfindungsgemäß ganz besonders bevorzugt sind Alkyloligoglucoside auf der Basis von gehärtetem C_{12/14}-Kokosalkohol mit einem DP von 1-3, wie sie beispielsweise unter der INCI-Bezeichnung "Coco-Glucoside" im Handel erhältlich sind.

In einer besonders bevorzugten Ausführungsform der Erfindung wird als Tensidbasis eine besonders milde Tensidmischung angestrebt, die sich für den Einsatz in kosmetischen, pflegenden Haut- und/oder Haarreinigungsmitteln eignet.
Für eine solche Tensidmischung ist es erfindungsgemäß bevorzugt, eine Mischung aus milden anionischen und milden amphoteren und/oder zwitterionischen Tensiden einzusetzen.
Beispiele für solche Mischungen sind beispielsweise Alkylethersulfate, Alkylethercarbonsäuren, Acylglutamate oder Sulfosuccinate sowie Alkylbetaine, Sulfobetaine, Alkylamidoalkylbetaine, Alkylamphoacetate und -propionate.
Besonders bevorzugt ist eine Mischung aus Alkylethersulfaten und Alkylamidoalkylbetainen. Diese Tenside werden in Mengen von 0,1 - 45 Gew.%, bevorzugt in Mengen von 1 - 30 Gew.% und ganz besonders bevorzugt in Mengen von 1 - 17,5 Gew.%, bezogen auf das gesamte Mittel, eingesetzt.

Neben den vorgenannten zwingenden erfindungsgemäßen Bestandteilen kann die Zusammensetzung eine Reihe weiterer fakultativer Bestandteile enthalten. Bevorzugt werden den Zusammensetzungen weitere Wirkstoffe zugesetzt, die kosmetische Pflegeeigenschaften aufweisen. Als solche sind insbesondere die kationischen Polymere, die Pflanzenextrakte und die Feuchthaltemittel als weitere bevorzugte fakultative Komponenten zu nennen.

Unter erfindungsgemäß geeigneten kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen. Homopolymere der allgemeinen Formel (VI), 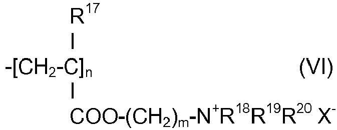 in der R¹⁷ = -H oder -CH₃ ist, R¹⁸, R¹⁹ und R²⁰ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁷ steht für eine Methylgruppe
- R¹⁸, R¹⁹ und R²⁰ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecylpolyoxypropylen-polyoxyethylen-ether (INCI-Bezeichnung: PPG-1-Trideceth-6)) im Handel erhältlich.
Copolymere mit Monomereinheiten gemäß Formel (VI) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer J^{R}® 400 sind bevorzugte quaternierte Cellulose-Derivate,
- hydrophob modifizierte Cellulosederivate, beispielsweise die unter dem Handelsnamen SoftCat^{®} vertriebenen kationischen Polymere,
- kationische Alkylpolyglycoside,
- kationiserter Honig, beispielsweise das Handelsprodukt Honeyquat^{®} 50,
- kationische Guar-Derivate, wie insbesondere die unter den Handelsnamen Cos-media^{®} Guar und Jaguar^{®} vertriebenen Produkte,
- Polysiloxane mit quaternären Gruppen, wie beispielsweise die im Handel erhältlichen erhältlichen Produkte Q2-7224 (Hersteller: Dow Corning; ein stabilisiertes Trimethylsilylamodimethicon), Dow Corning^{®} 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®} 100 (Poly(dimethyldiallylammoniumchlorid)) und Merquat^{®} 550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.
Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

In einer besonders bevorzugten Ausführungsform der Erfindung ist als kationisches Polymer mindestens ein Polymer aus der Gruppe der kationischen Guar-Derivate und/oder Polyquaternium-7 (Merquat 550), Polyquaternium-6, Polyquaternium-10 und/oder Polyquaternium-67 (SoftCat^{®}-Polymere) in den erfindungsgemäßen Zusammensetzungen enthalten.
Das oder die kationische(n) Polymer(e) ist (sind) in den erfindungsgemäßen Zusammensetzungen - bezogen auf die gesamte Zusammensetzung - in Mengen von 0,1 bis 5 Gew.-% enthalten. Mengen von 0,2 bis 3, insbesondere von 0,5 bis 2 Gew.-%, sind besonders bevorzugt.

Unter erfindungsgemäß geeigneten Pflanzenextrakten sind Extrakte zu verstehen, die aus allen Teilen einer Pflanze hergestellt werden können.
Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.
Erfindungsgemäß sind vor allem die Extrakte aus Grünem Tee, Eichenrinde, Brennessel, Hamamelis, Hopfen, Kamille, Klettenwurzel, Schachtelhalm, Weißdorn, Lindenblüten, Litschi, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Foeniculum vulgaris und Apim graveolens bevorzugt.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.
Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.
Weiterhin kann es bevorzugt sein Mischungen aus mehreren, insbesondere aus zwei, verschiedenen Pflanzenextrakten einzusetzen.

Zusätzlich kann es sich als vorteilhaft erweisen, wenn in den erfindungsgemäßen Zusammensetzungen Feuchthaltemittel bzw. Penetrationshilfsstoffe und/ oder Quellmittel (M) zuzusetzen. Diese Hilfsstoffe sorgen für eine bessere Penetration von Wirkstoffen in die keratinische Faser oder helfen die keratinische Faser aufzuquellen. Hierzu sind beispielsweise zu zählen Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol. Erfindungsgemäß besonders geeignet ist Glycerin.
Die Feuchthaltemittel werden in den erfindungsgemäßen Zusammensetzungen - bezogen auf die gesamte Zusammensetzung - in Mengen von 0,01 bis 10 Gew.-%, bevorzugt in Mengen von 0,05 bis 5 Gew.-% und insbesondere in Mengen von 0,1 bis 3 Gew.-% eingesetzt.

In einer besonders bevorzugten Ausführungsform der Erfindung wird die klare Zusammensetzung als kosmetische Zusammensetzung zur Reinigung der Haut und/oder der Haare, wie ein Haarshampoo, Duschgel, Duschbad, Waschgel, Gesichtsreiniger, Handwaschmittel und/oder ein Schaumbad konfektioniert. Als solche kann sie neben den vorgenannten zwingenden und bevorzugten fakultativen Komponenten noch weitere Wirk-, Hilfs- und Zusatzstoffe enthalten, die im folgenden beschrieben werden.

Neben den vorgenannten, erfindungsgemäß zwingenden und bevorzugten Inhaltsstoffen, können die tensidhaltigen Zusammensetzungen weiterhin die folgenden Komponenten enthalten: Erfindungsgemäß einsetzbar sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkyl-methylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.
Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats. Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.
Die kationischen Tenside werden bevorzugt in Mengen von 0,05 bis 10 Gew.-%, bezogen auf das gesamte Mittel, eingesetzt. Mengen von 0,1 bis 5 Gew.-% sind besonders bevorzugt.

In einer weiteren bevorzugten Ausführungsform kann die Wirkung der erfindungsgemäßen Zusammensetzungen durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, zum Beispiel das im Handel erhältliche Produkt Montanov^{®} 68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospholipide, die z.B. als Lecithine bzw. Phosphatidylcholine aus z.B. Eidotter oder Pflanzensamen (z.B. Sojabohnen) gewonnen werden, verstanden.
- Fettsäureester von Zuckern und Zuckeralkoholen wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C-Atomen und deren Na-, K-, Ammonium-, Ca-, Mg- und Zn - Salze.
Die Emulgatoren werden bevorzugt in Mengen von 0,1 - 25 Gew.-%, insbesondere 0,5 - 15 Gew.-%, bezogen auf das gesamte Mittel, eingesetzt.

Es kann zudem von Vorteil sein, wenn die erfindungsgemäßen Zusammensetzungen zusätzlich zu den kationischen Polymeren weitere natürliche und/oder synthetische Polymere, welche anionisch oder amphoter geladen sowie nichtionisch sein können, enthalten.
Bei den anionischen Polymeren handelt es sich üblicherweise um ein anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.
Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.
Besonders bevorzugt ist das Homopolymer der 2-Acrylamido-2-methylpropansulfonsäure, das beispielsweise unter der Bezeichnung Rheothik^{®}11-80 im Handel erhältlich ist.
Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.
Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylenbisacrylamid zum Einsatz kommen. Ein solches Polymer ist in dem Handelsprodukt Sepigel^{®} 305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.
Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.
Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.
Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnungg Stabileze^{®} QM im Handel erhältlich.
Weiterhin können als Polymere zur Steigerung der Wirkung des erfindungsgemäßen Mittels amphotere Polymere als Bestandteil eingesetzt werden. Unter dem Begriff amphotere Polymere werden sowohl solche Polymere, die im Molekül sowohl freie Aminogruppen als auch freie - COOH- oder SO₃H-Gruppen enthalten und zur Ausbildung innerer Salze befähigt sind, als auch zwitterionische Polymere, die im Molekül quartäre Ammoniumgruppen und -COO- oder -SO³⁻-Gruppen enthalten, und solche Polymere zusammengefaßt, die -COOH- oder SO₃H-Gruppen und quartäre Ammoniumgruppen enthalten.
Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.
Bevorzugt einsetzbare amphotere Polymere sind solche Polymerisate, die sich im wesentlichen zusammensetzen aus
(a) Monomeren mit quartären Ammoniumgruppen der allgemeinen Formel (PII),

   R²²-CH=CR²³-CO-Z-(CₙH₂ₙ)-N⁽⁺⁾R²⁴R²⁵R²⁶ A⁽⁻⁾ (PII)

   in der R²² und R²³ unabhängig voneinander stehen für Wasserstoff oder eine Methylgruppe und R²⁴, R²⁵ und R²⁶ unabhängig voneinander für Alkylgruppen mit 1 bis 4 KohlenstoffAtomen, Z eine NH-Gruppe oder ein Sauerstoffatom, n eine ganze Zahl von 2 bis 5 und A⁽⁻⁾ das Anion einer organischen oder anorganischen Säure ist
   und
(b) monomeren Carbonsäuren der allgemeinen Formel (PIII),

   R²⁷-CH=CR²⁸-COOH (PIII)

   in denen R²⁷ und R²⁸ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R²⁴, R²⁵ und R²⁶ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.
Als nichtionogene Polymere eignen sich erfindungsgemäß:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) vertrieben werden.
- Schellack
- Polyvinylpyrrolidone, wie sie beispielsweise unter der Bezeichnung Luviskol^{®} (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder HydroxyGruppen enthalten.
- Glycosidisch substituierte Silicone.
Der Einsatz der weiteren Polymere in Mengen von 0,01 bis 10 Gew.-%, bezogen auf das gesamte Mittel, ist erfindungsgemäß bevorzugt. Mengen von 0,1 bis 5 Gew.-%, insbesondere von 0,1 bis 3 Gew.-%, sind besonders bevorzugt.

In einer weiteren Ausführungsform der Erfindung können zur Steigerung der Wirkung der erfindungsgemäßen Zusammensetzung weiterhin Proteinhydrolysate und/oder deren Derivate eingesetzt werden. Proteinhydrolysate sind Produktgemische, die durch sauer, basisch oder enzymatisch katalysierten Abbau von Proteinen (Eiweißen) sowohl pflanzlichen als auch tierischen Ursprungs erhalten werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.
Erfindungsgemäß bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.
Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Gluadin^{®} (Cognis), Lexein^{®} (Inolex), Crolastin^{®} (Croda) oder Crotein^{®} (Croda) vertrieben.
Erfindungsgemäß einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden.
Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein. Ganz besonders bevorzugt sind die kationischen Proteinhydrolysate und -derivate auf pflanzlicher Basis.
Die Proteinhydrolysate und deren Derivate werden bevorzugt in Mengen von 0,01 - 10 Gew.-%, bezogen auf das gesamte Mittel, eingesetzt. Mengen von 0,1 bis 5 Gew.%, insbesondere 0,1 bis 3 Gew.-%, sind ganz besonders bevorzugt.

Ebenfalls als vorteilhaft hat sich die Kombination der erfindungsgemäßen Zusammensetzung mit Vitaminen, Provitaminen und Vitaminvorstufen sowie deren Derivaten erwiesen.
Dabei sind erfindungsgemäß solche Vitamine, Pro-Vitamine und Vitaminvorstufen bevorzugt, die üblicherweise den Gruppen A, B, C, E, F und H zugeordnet werden.
Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die Vitamin A-Komponente wird bevorzugt in Mengen von 0,05-1 Gew.-%, bezogen auf die gesamte Zubereitung, eingesetzt.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in bevorzugt in Mengen von 0,05 bis 1 Gew.-%, bezogen auf das gesamte Mittel, eingesetzt wird.
- Vitamin B₅ (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs werden bevorzugt in Mengen von 0,05- 10 Gew.-%, bezogen auf das gesamte Mittel, eingesetzt. Mengen von 0,1 - 5 Gew.-% sind besonders bevorzugt.
- Vitamin B₆ (Pyridoxin sowie Pyridoxamin und Pyridoxal).
Vitamin C (Ascorbinsäure). Die übliche Einsatzmenge von Vitamin C beträgt 0,1 bis 3 Gew.-%, bezogen auf das gesamte Mittel. Die Verwendung in Form des Palmitinsäureesters, der Glucoside oder Phosphate kann bevorzugt sein. Die Verwendung in Kombination mit Tocopherolen kann ebenfalls bevorzugt sein.
Vitamin E (Tocopherole, insbesondere α-Tocopherol). Tocopherol und seine Derivate, worunter insbesondere die Ester wie das Acetat, das Nicotinat, das Phosphat und das Succinat fallen, werden erfindungsgemäß bevorzugt in Mengen von 0,05-1 Gew.-%, bezogen auf das gesamte Mittel, eingesetzt.
Vitamin F. Unter dem Begriff "Vitamin F" werden üblicherweise essentielle Fettsäuren, insbesondere Linolsäure, Linolensäure und Arachidonsäure, verstanden.
Vitamin H. Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat. Biotin wird bevorzugt in Mengen von 0,0001 bis 1,0 Gew.-%, insbesondere in Mengen von 0,001 bis 0,01 Gew.-% eingesetzt.
Erfindungsgemäß besonders bevorzugt ist der Einsatz von Vitaminen, Provitaminen und Vitaminvorstufen aus den Gruppen A, B, E und H.
Panthenol und seine Derivate sowie Nicotinsäureamid und Biotin sind besonders bevorzugt.

Weiterhin kann in einer bevorzugten Ausführungsform der Erfindung zusätzlich ein UV - Filter (I) eingesetzt werden. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.
Die UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.
Gemäß einer weiteren Ausführungsform der Erfindung sind solche UV-Filter bevorzugt, die eine kationische Gruppe, insbesondere eine quartäre Ammoniumgruppe, aufweisen.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyl-dimethylaminobenzamidopropyl-dimethylammoniumtosylat (Escalol^{®} HP 610). Selbstverständlich umfasst die erfindungsgemäße Lehre auch die Kombination von mehreren UV-Filtern.
Die UV-Filter (I) werden üblicherweise in Mengen 0,1-5 Gew.-%, bezogen auf das gesamte Mittel, eingesetzt. Mengen von 0,4-2,5 Gew.-% sind bevorzugt.

Hinsichtlich der Art der erfindungsgemäßen kosmetischen Zubereitungen bestehen keine prinzipiellen Einschränkungen. Als Konfektionierung dieser Zubereitungen sind beispielsweise Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, grobe, instabile, ein oder mehrphasige Schüttelmixturen, Gele, Sprays, Aerosole und Schaumaerosole geeignet.

Weitere Wirk-, Hilfs- und Zusatzstoffe sind beispielsweise:
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn - Seifen,
- Strukturanten wie Maleinsäure und Milchsäure,
- Dimethylisosorbid,
- Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat,
- Farbstoffe zum Anfärben des Mittels,
- Antischuppenwirkstoffe wie Piroctone Olamine, Zink Omadine und Climbazol,
- weitere Substanzen zur Einstellung des pH-Wertes, wie beispielsweise α- und β-Hydroxycarbonsäuren,
- Wirkstoffe wie Allantoin und Bisabolol,
- Cholesterin,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Pigmente,
- Reduktionsmittel wie z. B. Thioglykolsäure und deren Derivate, Thiomilchsäure, Cysteamin, Thioäpfelsäure und α-Mercaptoethansulfonsäure,
- Treibmittel wie Propan-Butan-Gemische, N₂O, Dimethylether, CO₂ und Luft,
- Antioxidantien,
- Desoxyzucker, Pflanzenglycoside, Polysaccharide wie Fucose oder Rhamnose,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Hydrogencarbonate, Guanidine, Harnstoffe sowie primäre, sekundäre und tertiäre Phosphate,
- Konservierungsmittel, wie beispielsweise Natriumbenzoat,
- Viskositätsregler wie Salze (NaCl).
Ein zweiter Gegenstand der Erfindung ist Verwendung einer Kombination aus Lösungsvermittlern, die ausgewählt sind aus der Gruppe der
a) ethoxylierten Fettalkohole,
b) ethoxylierten hydrierten Rizinusöle und
c) ethoxylierten Mono-, Di- oder Triglycerinester,
in einem Verhältnis der Komponenten a): b): c) im Bereich von 1 : (2-4) : (3-4), zur Suspendierung und Stabilisierung einer Ölkomponente in einer klaren tensidhaltigen Zusammensetzung und zur Herstellung einer Zusammensetzung mit einer stabilen, konstanten Viskosität im Bereich von 5,000 bis 9,500 mPas.
Ein dritter Gegenstand der Erfindung ist die Verwendung der erfindungsgemäßen Zusammensetzung zur Reinigung und Pflege menschlicher Haut und menschlicher Haare.
Ein vierter Gegenstand der Erfidnung ist ein Verfahren zur Suspendierung und Stabilisierung einer Ölkomponente in einer tensidhaltigen Zusammensetzung, dadurch gekennzeichnet, dass eine Mischung aus Lösungsvermittlern, die ausgewählt sind aus
a) ethoxylierten Fettalkoholen,
d) ethoxylierten hydrierten Rizinusölen und
e) ethoxylierten Mono-, Di- oder Triglycerinestern,
in einem Verhältnis der Komponenten a): b): c) im Bereich von 1 : (2-4) : (3-4) bei Raumtemperatur gerührt wird, bis eine klare Lösung entsteht, anschließend zunächst mit Ölkomponenten versetzt und gerührt wird, bis eine klare Lösung entsteht, und dann mit dem Verdickungsmittel versetzt wird, bevor die klare Mischung in eine tensidhaltige Basis eingearbeitet wird.
Die folgenden Beispiele erläutern die Erfindung, ohne sie jedoch darauf einzuschränken:
Alle Angaben sind - sofern nicht anders angegeben - in Gewichtsprozent.

### Beispiele

| **Rohstoff** | **1** | **2** | **3** | **4** | **5** | **6** | **7** | **8** | **9** | **10** | **11** | **12** | **13** | **14** |
|---|---|---|---|---|---|---|---|---|---|---|---|---|---|---|
| **(SLES), 2 EO, 70%** | 10 | 10 | 10 | 10 | 10 | 10 | 10 | 10 | 10 | 10 | 10 | 10 | 10 | 10 |
| **CAPB 40%** | 10 | 10 | 10 | 10 | 10 | 10 | 10 | 10 | 10 | 10 | 10 | 10 | 10 | 10 |
| **Conditioner^{®1} P7** | 1,5 | 1,5 | 1,5 | 1,5 | 1,5 | 1,5 | 1,5 | 1,5 | 1,5 | 1,5 | 1,5 | 1,5 | 1,5 | 1,5 |
| **Glycerin 86%** | 1 | 1 | 1 | 1 | 1 | 1 | 1 | 1 | 1 | 1 | 1 | 1 | 1 | 1 |
| **Herbasol-Extrakt Litschi** | 0,1 | 0,1 | 0,1 | 0,1 | 0,1 | 0,1 | 0,1 | 0,1 | 0,1 | 0,1 | 0,1 | 0,1 | 0,1 | 0,1 |
| **Natriumbenzoat** | 0,4 | 0,4 | 0,4 | 0,4 | 0,4 | 0,4 | 0,4 | 0,4 | 0,4 | 0,4 | 0,4 | 0,4 | 0,4 | 0,4 |
| **Citronensäure** | 0,2 | 0,2 | 0,2 | 0,2 | 0,2 | 0,2 | 0,2 | 0,2 | 0,2 | 0,2 | 0,2 | 0,2 | 0,2 | 0,2 |
| **Mandelöl, süß** | 0,2 | 0,2 | 0,2 | 0,2 | 0,2 | 0,2 | 0,2 | 0,2 | 0,2 | 0,2 | 0,2 | 0,2 | 0,2 | 0,2 |
| **Parfum** | 1 | 1 | 1 | 1 | 1 | 1 | 1 | 1 | 1 | 1 | 1 | 1 | 1 | 1 |
| **Lösungsvermittler 660352^{®2}** | | 1 | 1 | 1 | | | | | | | 1 | 0,5 | 0,5 | 0,5 |
| **Cetiol**^{®}**³ HE** | 1 | | 1 | 1 | 1 | 1 | 0,5 | 0,7 | 1 | 1 | 1 | 1 | 1 | 0,8 |
| **Eumulgin^{®4} HRE 455** | 0,3 | | | 0,3 | 0,2 | 0,5 | | | | | 0,2 | 0,2 | 0,4 | 0,4 |
| **Eumulgin^{®5} HRE 40** | | | | | | | 0,3 | 0,3 | 0,2 | 0,3 | | | | |
| **NaCl** | 0,9 | 0,9 | 0,2 | | 1 | 1 | | 1 | 1,2 | 1,2 | 0,2 | 0,2 | 0,2 | 0,4 |
| **Antil^{®6} 141** | 0,2 | 0,2 | 0,6 | 1 | | | | | 0,5 | 0,5 | 0,5 | 0,5 | 0,5 | 0,6 |
| **Arlypon^{®7} F** | 0,2 | 0,2 | | | 0,5 | | | | | | | | | 0,6 |
| **Wasser** | ad 100 | ad 100 | ad 100 | ad 100 | ad 100 | ad 100 | ad 100 | ad 100 | ad 100 | ad 100 | ad 100 | ad 100 | ad 100 | ad 100 |
| **Viskosität in Pas** | 3,2 | 2,6 | | | | | | | 7,4 | 6,8 | | | | 7,9 |
| **Aussehen** | **k** | **k** | **t** | **t** | **lt** | **k** | **t** | **t** | **k** | **k** | **t** | **lt** | **k** | **k** |
| **3 Tage (60°C Lagerung)** | **k** | **lt** | | | | **k** | | | **t** | **t** | | | | **k** |
| **7 Tage (50°C Lagerung)** | | | | | | | | | **t** | **t** | | | | **k** |
| **3 Tage (Raumtemp.)** | **k** | **lt** | | | | **k** | | | | | | | | |
| **>3 Tage** | **t** | | | | | | | | | | **t** | **lt** | **lt** | **k** |

| | | | | | | | | | | | | | | |
|---|---|---|---|---|---|---|---|---|---|---|---|---|---|---|
| **t = trüb; k= klar; lt= leicht trüb** | | | | | | | | | | | | | | |

Aus der Spalte 14 der Tabelle wird ersichtlich, dass nur die erfindungsgemäßen Zusammensetzungen alle Erfordernisse hinsichtlich der Viskositäts- und der Suspensionsstabilität erfüllen.
Es wurden die folgenden Handelsprodukte eingesetzt:
SLES: INCI-Bezeichnung: Sodium Laureth Sulfate (2 EO); 68-75% AS; Cognis
CAPB: INCI-Bezeichnung: Cocamidopropyl Betaine; ca. 44% AS; Cognis
1 INCI-Bezeichnung: Polyquaternium-7, Aqua; Sigma
2 INCI-Bezeichnung: PEG-40 Hydrogenated Castor Oil, Trideceth-9, Propylene Glycol, Aqua; Symrise
3 INCI-Bezeichnung: PEG-7 Glyceryl Cocoate; Cognis
4 INCI-Bezeichnung: PEG-40 Hydrogenated Castor Oil, Propylene Glycol, Aqua; Cognis
5 INCI-Bezeichnung: PEG-40 Hydrogenated Castor Oil; Cognis
6 INCI-Bezeichnung: Propylene Glycol, PEG-55 Propylene Glycol Oleate; Goldschmidt
7 INCI-Bezeichnung: Laureth-2; Cognis

## Patentansprüche

1. Klare tensidhaltige Zusammensetzung, enthaltend neben einer oder mehrerer Ölkomponente(n) ein Gemisch aus Lösungsvermittlern, die ausgewählt sind aus der Gruppe der
a) ethoxylierten Fettalkohole,
b) ethoxylierten hydrierten Rizinusöle und
c) ethoxylierten Mono-, Di- oder Triglycerinester,
**dadurch gekennzeichnet, dass** das Verhältnis der Komponenten a) : b) : c) im Bereich von 1 : (2-4): (3-4) liegt.

2. Zusammensetzung nach Anspruch 1, **dadurch gekennzeichnet, dass** sie einen NTU-Wert (Nephelometric Turbidity Unit) ≤ 50, bevorzugt ≤ 30, besonders bevorzugt ≤ 20 und insbesondere ≤ 10 aufweist.

3. Zusammensetzung nach einem der Ansprüche 1 oder 2, **dadurch gekennzeichnet, dass** die Komponente a) ausgewählt ist aus geradkettigen oder verzweigten, gesättigten oder ungesättigten C₈-C₂₂-Fettalkoholen, bevorzugt aus C₁₀-C₂₀-Fettalkoholen, besonders bevorzugt aus C₁₂-C₁₈-Fettalkoholen und insbesondere aus C₁₃-C₁₅-Fettalkoholen mit einem Ethoxylierungsgrad von 1 bis 18, bevorzugt von 5 bis 16, besonders bevorzugt von 7 bis 14 und insbesondere von 8 bis 12.

4. Zusammensetzung nach einem der Ansprüche 1 bis 3, **dadurch gekennzeichnet, dass** die Komponente b) einen Ethoxylierungsgrad von 5 bis 80, bevorzugt von 10 bis 60, besonders bevorzugt von 25 bis 50 und insbesondere von 35 bis 45 aufweist.

5. Zusammensetzung nach einem der Ansprüche 1 bis 4, **dadurch gekennzeichnet, dass** die Komponente c) ausgewählt ist aus Monoestern und/oder Gemischen aus Monoestern und Diestern des Glycerins mit verzweigten, oder geradkettigen, gesättigten oder ungesättigten Fettsäuren einer C-Kettenlänge von 8 bis 24, bevorzugt von 10 bis 18 und insbesondere von 12 bis 16, die einen Ethoxylierungsgrad von 1 bis 20, bevorzugt von 2 bis 17, besonders bevorzugt von 4 bis 13 und insbesondere von 6 bis 10 aufweisen.

6. Zusammensetzung nach einem der Ansprüche 1 bis 5, **dadurch gekennzeichnet, dass** sie die Komponenten a), b) und c) - bezogen auf ihr Gesamtgewicht - jeweils in Mengen von 0,05 bis 3 Gew.-%, bevorzugt von 0,1 bis 2 Gew.-% und insbesondere von 0,2 bis 1,5 Gew.-% enthält.

7. Zusammensetzung nach einem der Ansprüche 1 bis 6, **dadurch gekennzeichnet, dass** sie eine Viskosität im Bereich von 5,000 bis 9,500 mPas, bevorzugt von 6,000 bis 9,000 mPas und insbesondere von 7,000 bis 8,000 mPas (jeweils gemessen mit einem Haake Rotationsviskosimeter VT550; 20°C; Meßeinrichtung MV; Spindel MV II; 8 UPM) aufweist.

8. Zusammensetzung nach einem der Ansprüche 1 bis 7, **dadurch gekennzeichnet, dass** die Ölkomponente(n) ausgewählt ist (sind) aus mineralischen, natürlichen oder synthetischen Ölkomponenten wie Petrolatum, Paraffinen, Silikonen, Fettalkoholen, Fettsäuren, Fettsäureestern sowie natürlichen Ölen pflanzlichen und tierischen Ursprungs, die - bezogen auf das Gesamtgewicht der Zusammensetzung - in einer Menge von 0,005 bis 20 Gew.-%, bevorzugt von 0,01 bis 10 Gew.-%, besonders bevorzugt von 0,05 bis 5 Gew.-% und insbesondere von 0,2 bis 3 Gew.-% enthalten ist (sind).

9. Zusammensetzung nach Anspruch 8, **dadurch gekennzeichnet, dass** sie als Ölkomponente natürliche pflanzliche Öle enthält.

10. Zusammensetzung nach einem der Ansprüche 1 bis 9, **dadurch gekennzeichnet, dass** sie eine milde Tensidmischung aus mindestens einem anionischen und mindestens einem amphoteren/zwitterionischen Tensid enthält.

11. Zusammensetzung nach einem der Ansprüche 1 bis 10, **dadurch gekennzeichnet, dass** sie weiterhin mindestens einen weiteren Wirkstoff enthält, der ausgewählt ist aus der Gruppe der kationischen Polymere, der Pflanzenextrakte und der Feuchthaltemittel.

12. Zusammensetzung nach einem der Ansprüche 1 bis 11, **dadurch gekennzeichnet, dass** sie als kosmetische Zusammensetzung zur Reinigung der Haut und/oder der Haare, wie ein Haarshampoo, Duschgel, Duschbad, Waschgel, Gesichtsreiniger, Handwaschmittel und/oder ein Schaumbad konfektioniert ist.

13. Verwendung einer Kombination aus Lösungsvermittlern, die ausgewählt sind aus der Gruppe der
a) ethoxylierten Fettalkohole,
b) ethoxylierten hydrierten Rizinusöle und
c) ethoxylierten Mono-, Di- oder Triglycerinester,
in einem Verhältnis der Komponenten a): b) : c) im Bereich von 1 : (2-4) : (3-4), zur Suspendierung und Stabilisierung einer Ölkomponente in einer klaren tensidhaltigen Zusammensetzung und zur Herstellung einer Zusammensetzung mit einer stabilen, konstanten Viskosität im Bereich von 5,000 bis 9,500 mPas.

14. Verwendung einer Zusammensetzung nach einem der Ansprüche 1 bis 11 zur Reinigung und Pflege menschlicher Haut und menschlicher Haare.

15. Verfahren zur Suspendierung und Stabilisierung einer Ölkomponente in einer tensidhaltigen Zusammensetzung, **dadurch gekennzeichnet, dass** eine Mischung aus Lösungsvermittlern, die ausgewählt sind aus
a) ethoxylierten Fettalkoholen,
b) ethoxylierten hydrierten Rizinusölen und
c) ethoxylierten Mono-, Di- oder Triglycerinestern,
in einem Verhältnis der Komponenten a): b): c) im Bereich von 1 : (2-4) : (3-4) bei Raumtemperatur gerührt wird, bis eine klare Lösung entsteht, anschließend zunächst mit Ölkomponenten versetzt und gerührt wird, bis eine klare Lösung entsteht, und dann mit dem Verdickungsmittel versetzt wird, bevor die klare Mischung in eine tensidhaltige Basis eingearbeitet wird.

## Claims

1. A clear surfactant-containing composition, in addition to one or more oil components comprising a mixture of solubilizers selected from the group of
a) ethoxylated fatty alcohols,
b) ethoxylated hydrogenated castor oils, and
c) ethoxylated mono-, di- or triglycerol esters,
**characterized in that** the ratio of components a):b):c) is in the range of 1:(2-4):(3-4).

2. The composition according to claim 1, **characterized by** having an NTU (Nephelometric Turbidity Unit) value of ≤ 50, preferably ≤ 30, particularly preferably ≤ 20, and in particular ≤ 10.

3. The composition according to either claim 1 or 2, **characterized in that** component a) is selected from straight-chain or branched, saturated or unsaturated C₈ to C₂₂ fatty alcohols, preferably from C₁₀ to C₂₀ fatty alcohols, particularly preferably from C₁₂ to C₁₈ fatty alcohols, and in particular from C₁₃ to C₁₅ fatty alcohols having a degree of ethoxylation of 1 to 18, preferably 5 to 16, particularly preferably 7 to 14, and in particular 8 to 12.

4. The composition according to any one of claims 1 to 3, **characterized in that** component b) has a degree of ethoxylation of 5 to 80, preferably 10 to 60, particularly preferably 25 to 50, and in particular 35 to 45.

5. The composition according to any one of claims 1 to 4, **characterized in that** component c) is selected from monoesters and/or mixtures of monoesters and diesters of glycerol having branched or straight-chain, saturated or unsaturated fatty acids having a carbon chain length of 8 to 24, preferably 10 to 18, and in particular 12 to 16, which have a degree of ethoxylation of 1 to 20, preferably 2 to 17, particularly preferably 4 to 13, and in particular 6 to 10.

6. The composition according to any one of claims 1 to 5, **characterized by** comprising components a), b) and c) in each case in quantities of 0.05 to 3 wt.%, preferably 0.1 to 2 wt.%, and in particular 0.2 to 1.5 wt.%, based on the total weight of the composition.

7. The composition according to any one of claims 1 to 6, **characterized by** having a viscosity in the range of 5000 to 9500 mPas, preferably 6000 to 9000 mPas, and in particular 7000 to 8000 mPas (in each case measured using a Haake rotational viscometer VT500; 20 °C; measuring device MV; spindle MV II; 8 rpm).

8. The composition according to any one of claims 1 to 7, **characterized in that** the oil component(s) is (are) selected from mineral, natural or synthetic oil components, such as petroleum jelly, paraffins, silicones, fatty alcohols, fatty acids, fatty acid esters, and natural oils of plant and vegetable origin, which is (are) present in a quantity of 0.005 to 20 wt.%, preferably 0.01 to 10 wt.%, particularly preferably 0.05 to 5 wt.%, and in particular 0.2 to 3 wt.%, based on the total weight of the composition.

9. The composition according to claim 8, **characterized by** comprising natural vegetable oils as the oil component.

10. The composition according to any one of claims 1 to 9, **characterized by** comprising a mild surfactant mixture composed of at least one anionic surfactant and at least one amphoteric/zwitterionic surfactant.

11. The composition according to any one of claims 1 to 10, **characterized by** further comprising at least one further active ingredient selected from the group of cationic polymers, plant extracts, and humectants.

12. The composition according to any one of claims 1 to 11, **characterized by** being formulated as a cosmetic composition for cleaning the skin and/or hair, such as a hair shampoo, shower gel, shower soap, washing gel, facial cleanser, hand washing agent and/or a foam bath.

13. Use of a combination of solubilizers selected from the group of
a) ethoxylated fatty alcohols,
b) ethoxylated hydrogenated castor oils, and
c) ethoxylated mono-, di- or triglycerol esters,
in a ratio of components a):b):c) in the range of 1:(2-4):(3-4) for suspending and stabilizing an oil component in a clear surfactant-containing composition and for producing a composition having a stable, constant viscosity in the range of 5000 to 9500 mPas.

14. Use of a composition according to any one of claims 1 to 11 for cleaning and caring for human skin and human hair.

15. A method for suspending and stabilizing an oil component in a surfactant-containing composition, **characterized in that** a mixture of solubilizers selected from
a) ethoxylated fatty alcohols,
b) ethoxylated hydrogenated castor oils, and
c) ethoxylated mono-, di- or triglycerol esters,
in a ratio of components a):b):c) in the range of 1:(2-4):(3-4) is stirred at room temperature until a clear solution is obtained, subsequently is first mixed with oil components and stirred until a clear solution is created, and then is mixed with a thickening agent before the clear mixture is incorporated into a surfactant-containing base.

## Revendications

1. Composition claire contenant des tensioactifs, comportant en plus d'un ou de plusieurs composés oléiques un mélange d'agents de solubilisation qui sont choisis dans le groupe
a) des alcools gras éthoxylés,
b) des huiles de ricin hydrogénées éthoxylées et
c) des mono-, di- ou triglycérols éthoxylés,
**caractérisée en ce que** le rapport des composants a):b):c) est dans la gamme de 1:(2 à 4):(3 à 4).

2. Composition selon la revendication 1, **caractérisée en ce qu'**elle a une valeur UNT (unité néphélométrique de turbidité) < 50, de préférence < 30, de manière particulièrement préférée < 20, et en particulier < 10.

3. Composition selon l'une des revendications 1 ou 2, **caractérisée en ce que** le composé a) est choisi parmi les alcools gras en C₈ à C₂₂ linéaires ou ramifiés, saturés ou insaturés, de préférence les alcools gras en C₁₀ à C₂₀, de façon particulièrement préférée les alcools gras en C₁₂ à C₁₈ et en particulier les alcools gras en C₁₃ à C₁₅ ayant un degré d'éthoxylation de 1 à 18, de préférence de 5 à 16, plus préférentiellement de 7 à 14 et en particulier de 8 à 12.

4. Composition selon l'une des revendications 1 à 3, **caractérisée en ce que** le composé b) a un degré d'éthoxylation de 5 à 80, de préférence de 10 à 60, plus préférentiellement de 25 à 50 et en particulier de 35 à 45.

5. Composition selon l'une des revendications 1 à 4, **caractérisée en ce que** le composé c) est choisi parmi les mono-esters et/ou les mélanges de monoesters et de diesters de glycérol comportant des acides gras linéaires ou ramifiés, saturés ou insaturés ayant une longueur de chaîne carbonée de 8 à 24, de préférence de 10 à 18 et en particulier de 12 à 16, qui ont un degré d'éthoxylation de 1 à 20, de préférence de 2 à 17, plus préférentiellement de 4 à 13 et en particulier de 6 à 10.

6. Composition selon l'une des revendications 1 à 5, **caractérisée en ce qu'**elle contient les composés a), b) et c), sur la base de son poids total, à chaque fois dans des quantités allant de 0,05 à 3% en poids, de préférence de 0,1 à 2% en poids, et en particulier de 0,2 à 1,5% en poids.

7. Composition selon l'une des revendications 1 à 6, **caractérisée en ce qu'**elle présente une viscosité dans la gamme de 5000 à 9500 mPas, de préférence de 6000 à 9000 mPas et en particulier de 7000 à 8000 mPas (mesurée à chaque fois avec un viscosimètre rotatif Haake VT550 ; 20°C ; dispositif de mesure MV ; broche MV II ; 8 tours par minute).

8. Composition selon l'une des revendications 1 à 7, **caractérisée en ce que** le ou les composés oléiques sont choisis qui les composés oléiques minéraux, naturels ou synthétiques tels que la vaseline, les paraffines, les silicones, les alcools gras, les acides gras, les esters d'acides gras et les huiles naturelles d'origine végétale et animale qui sont contenus, sur la base du poids total de la composition, dans une quantité allant de 0,005 à 20% en poids, de préférence de 0,01 à 10% en poids, plus préférentiellement de 0,05 à 5% en poids et en particulier de 0,2 à 3% en poids.

9. Composition selon la revendication 8, **caractérisée en ce qu'**elle contient, comme composés oléiques, des huiles végétales naturelles.

10. Composition selon l'une des revendications 1 à 9, **caractérisée en ce qu'**elle contient un mélange de tensioactifs doux constitué d'au moins un tensioactif anionique et d'au moins un tensioactif amphotère/zwitterionique.

11. Composition selon l'une des revendications 1 à 10, **caractérisée en ce qu'**elle contient en outre au moins une autre substance active choisie dans le groupe des polymères cationiques, des extraits de plantes et des agents humidificateurs.

12. Composition selon l'une des revendications 1 à 11, **caractérisée en ce qu'**elle est conditionnée sous la forme d'une composition cosmétique servant à nettoyer la peau et/ou les cheveux, tel qu'un shampooing, un gel de douche, un savon de bain/douche, un gel nettoyant, un nettoyant pour le visage, un savon pour les mains et/ou un bain moussant.

13. Utilisation d'une combinaison d'agents de solubilisation qui sont choisis dans le groupe
a) des alcools gras éthoxylés,
b) des huiles de ricin hydrogénées éthoxylées et
c) des mono-, di- ou triglycérols éthoxylés,
dans un rapport des composés a):b):c) dans la gamme de 1:(2 à 4):(3 à 4), pour suspendre et stabiliser un composé oléique dans une composition claire contenant des tensioactifs et pour préparer une composition présentant une viscosité stable et constante dans la gamme de 5000 à 9500 mPas.

14. Utilisation d'une composition selon l'une des revendications 1 à 11 pour le nettoyage et les soins de la peau humaine et des cheveux humains.

15. Procédé de suspension et de stabilisation d'un composé oléique dans une composition contenant des tensioactifs, **caractérisée en ce qu'**un mélange d'agents de solubilisation, qui sont choisis parmi
a) les alcools gras éthoxylés,
b) les huiles de ricin hydrogénées éthoxylées et
c) les mono-, di- ou triglycérols éthoxylés,
dans un rapport des composés a):b):c) dans la gamme de 1:(2 à 4):(3 à 4), est agité à la température ambiante jusqu'à obtenir une solution claire, puis mélangé à des composés oléiques et agité jusqu'à obtenir une solution limpide, et ensuite mélangé à l'épaississant avant d'incorporer le mélange clair à une base contenant des tensioactifs.
